# EUROPÄISCHE PATENTSCHRIFT

(11) **EP 2 438 934 B1**
(45) Veröffentlichungstag und Bekanntmachung des Hinweises auf die Patenterteilung: **16.12.2015**
(21) Anmeldenummer: 11008056.1
(22) Anmeldetag: 05.10.2011
(51) Int. Cl.: A61M 1/00

(54) **Gelenkdruckmessung mit Sensor in der Drainageleitung**
Joint pressure measurement with sensor in the drainage conduit
Mesure de la pression d'articulation à l'aide d'un capteur dans la conduite de drainage

(30) Priorität: 05.10.2010 DE 102010047349
(43) Veröffentlichungstag der Anmeldung: 11.04.2012
(73) Patentinhaber: W.O.M. World of Medicine GmbH, 10587 Berlin (DE)
(72) Erfinder: Reuther, Martin, 96231 Bad Staffelstein (DE); Stiller, Matthias, 14482 Potsdam (DE); Merzhäuser, Thomas, 14469 Potsdam (DE)
(74) Vertreter: Seuss, Thomas

(56) Entgegenhaltungen:
- EP-A2- 1 382 291
- US-A- 4 940 457
- US-A- 5 556 378
- US-A1- 2002 068 913
- US-A1- 2003 236 488
- US-A1- 2008 154 184
- US-A1- 2008 154 185
- US-A1- 2009 163 852

## Beschreibung

### Gebiet der Erfindung

Die Erfindung betrifft eine Vorrichtung zum Durchspülen einer Körperhöhle mit einem Fluid, mit einem Vorratsbehälter für das Fluid, mit einer an den Vorratsbehälter angeschlossenen, ansteuerbaren Spülpumpe, mit einer an der Druckseite der Spülpumpe (3) angeschlossenen Zuführleitung sowie einem an die Zuführleitung angeschlossenen ersten ärztlichen Instruments mit einem Spülkanal, welches in die Körperhöhle (1) einführbar ist, mit einer in die Körperhöhle (1) einführbaren Drainagekanüle (7) mit Drainageleitung (8) wobei in oder an der Drainageleitung (8) ein Drucksensor (10) zur Bestimmung des Ist-Druckes eingerichtet ist, wobei die Drainageleitung (8) an eine Saugpumpe (9) angeschlossen ist, ein Verfahren zum Betrieb einer solchen Vorrichtung sowie die erfindungsgemäße Verwendung einer solchen Vorrichtung.

Solche Vorrichtungen werden beispielsweise für endoskopische Untersuchungen, Distension oder Resektion von Geweben, insbesondere unter endoskopische Kontrolle, eingesetzt. Eine Körperhöhle (1) kann ein Gelenkhohlraum, z.B. in einem Kniegelenk oder Schultergelenk, ein Hohlraum zwischen Muskeln oder Organen, oder auch ein einen hohlraumbildendes oder aufweisendes Organ selbst sein. Ein Fluid ist insbesondere eine Flüssigkeit. Dabei kann es sich um eine homogene flüssige Phase, beispielsweise wässrige Kochsalzlösung, oder auch um eine Dispersion oder Emulsion handeln. Eine Drainagekanüle (7) im Sinne der Erfindung kann neben der Drainagefunktion an sich auch weitere Funktionen erfüllen, wie beispielsweise ein Ausleuchten der Körperhöhle (1) über eine in die Drainagekanüle (7)integrierte Lichtquelle. Unterschiedliche Volumenströme bei unterschiedlichen Betriebsstellungen sind im Rahmen der Erfindung durch die Begriffe "hoch" und "niedrig" unterschieden, wobei die absoluten Werte irrelevant sind. Vielmehr wird mit diesen Begriffen lediglich zum Ausdruck gebracht, wie die Volumenströme relativ zueinander bei den jeweiligen Betriebsstellungen des zweiten ärztlichen Instrumentes sind.

### Stand der Technik

Gattungsgemäße Vorrichtungen sind beispielsweise aus den Literaturstellen US 4,902,277, US 5,000,733, EP 0306445, EP 1382291 oder US 6,024,720 bekannt. Als Schwierigkeit bei den bestehenden Vorrichtungen hat sich die Messung des Drucks in der Körperhöhle (1) erwiesen. Dieser Wert ist deswegen kritisch, weil ein zu hoher Druck zu irreversiblen Gewebeschädigungen führen kann. Im Rahmen der bekannten Vorrichtungen wird beispielsweise ein Drucksensor zusätzlich zur Zuführleitung, Abführleitung und ärztlichem Instrument in die Körperhöhle (1) eingeführt, was zu einer Erhöhung des Infektionsrisikos führt, aber auch zu Behinderungen der Arbeit führen kann. In alternativen Ausführungsformen ist ein Drucksensor druckseitig an der Zuführpumpe vorgesehen (siehe z. B. EP 1382291). Es hat sich allerdings herausgestellt, dass die Druckmessung deswegen schwierig ist, weil der in der Zuführleitung gemessene Druck vom Durchflusswiderstand des ersten ärztlichen Instrumentes abhängt. In der Praxis müssen daher Korrekturen des gemessenen Druckes erfolgen, wobei die Korrekturfaktoren von der Wahl des ärztlichen Instrumentes abhängen. Dies führt zu Problemen bei der Anwendung dieser Vorrichtungen.

### Technisches Problem der Erfindung

Der Erfindung liegt daher das technische Problem zugrunde, eine Vorrichtung zum Durchspülen einer Körperhöhle (1) mit einem Fluid anzugeben, welches eine Druckmessung in der Körperhöhle (1) erlaubt, ohne dass zusätzliche Zuführleitungen nötig sind und ohne dass die Druckmessung von den verwendeten ärztlichen Instrumenten abhängt.

### Grundzüge der Erfindung und bevorzugte Ausführungsformen

Zur Lösung dieses technischen Problems lehrt die vorliegende Erfindung eine Vorrichtung zum Durchspülen einer Körperhöhle (1) mit einem Fluid, mit einer ansteuerbaren Spülpumpe (3) mit einer an die Druckseite der Spülpumpe (3) angeschlossenen Zuführleitung (4), mit einem ersten ärztlichen Instrument (5),
mit einem zweiten ärztlichen Instrument (6), welches ein Shaver ist,
mit einer Drainagekanüle (7), angeschlossen an eine Drainageleitung (8) mit einem Quetschventil (11),
mit einer optionalen zweiten Pumpe (9) zum Absaugen des Fluids aus der Drainageleitung (8),
wobei,
an der Drainageleitung (8) vor dem Quetschventil (11) ein erster Drucksensor (10) enthalten ist, der den Druck in der Körperhöhle (1) bestimmt
und wobei ein zweiter Drucksensor (12) an der Druckseite der Spülpumpe (3) an der Zuführleitung angeschlossen ist und wobei
die Druckmessung mit dem Drucksensor (10) erfolgt, wenn das Quetschventil (11) geschlossen ist und dass die Druckmessung mit dem Drucksensor (12) erfolgt, wenn das Quetschventil (11) offen ist.

Ein essentielles Element der vorliegenden Erfindung besteht darin, einen Drucksensor (10) ("erster Drucksensor") in der Drainageleitung (8) vorzusehen. Die Drainageleitung (8) weist darüber hinaus ein Quetschventil (pinch valve, 11) auf, das die Drainageleitung (8) von der Saugseite der Saugpumpe (9) absperren kann. Zur Bestimmung des Drucks in der Körperhöhle, wird das Quetschventil geschlossen und der Drucksensor (10) zeigt unmittelbar den Druck in der Körperhöhle (1) an.

Die Spülpumpe (3) kann eine Antriebseinheit mit einem Motor mit rotierender Antriebswelle sowie eine mit der Antriebswelle (mechanisch), insbesondere stoffschlüssig, formschlüssig oder kraftschlüssig, oder magnetisch insbesondere kraftschlüssig, verbundene und rotierend angetriebene Pumpeneinheit aufweisen, wobei die Förderleistung durch Ansteuerung der Drehzahl des Motors ansteuerbar ist. Insbesondere kommt insofern eine peristaltische Rollenpumpe infrage. Alternativ kann die Spülpumpe (3)einen elastischen Vorratsbehälter mit einer den Vorratsbehälter teilweise oder ganz ummantelnden ansteuerbaren Druckmanschette aufweisen, wobei die Förderleistung durch Ansteuerung der Druckmanschette ansteuerbar ist. Schließlich kann weiterhin alternativ die Spülpumpe (3)einen höhenansteuerbaren Vorratsbehälter aufweisen, wobei die Förderleistung durch Ansteuerung der Höhe des Vorratsbehälters ansteuerbar ist.

Für die Saugpumpe kommt ebenfalls insbesondere eine peristaltische Rollenpumpe infrage.

Das erste ärztliche Instrument kann ausgewählt sein aus der Gruppe bestehend aus Spülsonde, Trokare mit Optik, Optik mit Spülkanüle, Spülkanüle, Shaver. Bei dem erfindungsgemäßen Quetschventil (11) handelt es sich um eine Schlauchabklemmvorrichtung, wobei die Schlauchwandung aus einem elastischen Werkstoff besteht und das Ventil eine Stützfläche enthält, auf welcher die Schlauchwandung des Schlauches aufliegt, und ein Druckstück enthält, mittels welchem auf die Schlauchwandung Druck in Richtung der Stützfläche ausgeübt werden kann. Bevorzugt ist, dass das Druckstück linear antreibbar und über ein Spindelgetriebe mit einem elektromotorischen Antrieb, vorzugsweise einem Schrittschaltmotor, verbunden ist.

Die Drainageleitung (8), bzw. die Saugpumpe(9) mündet letztendlich in ein Auffanggefäß (12).

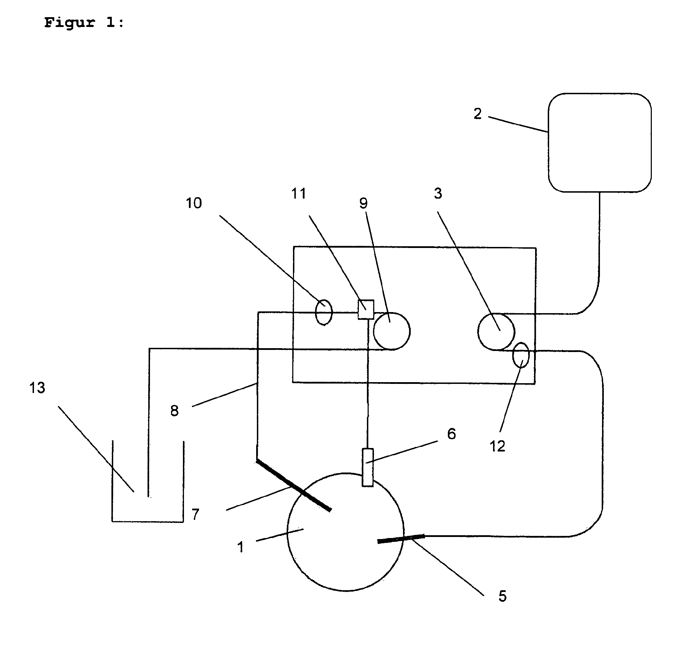

Im Folgenden wird die Erfindung anhand der Figur 1 näher erläutert:
In der Figur 1 erkennt man eine Vorrichtung zum Durchspülen einer Körperhöhle (1) mit einem Fluid. Diese Vorrichtung ist mit einem Vorratsbehälter (2) für das Fluid und mit einer an den Vorratsbehälter (2) angeschlossenen ansteuerbaren Spülpumpe (3)ausgestattet. An der Druckseite der Spülpumpe (3) ist eine Zuführleitung (4) angeschlossen. Die Zuführleitung ist mit einem ersten ärztlichen Instrument mit einem Spülkanal (5), welches in die Körperhöhle (1) einführbar ist, versehen. Druckseitig an der Spülpumpe (3) ist ein zusätzlicher Drucksensor (12) zur Bestimmung eines Ist-Druckes eingerichtet.

Weiterhin erkennt man eine Drainageleitung (8), die in die Körperhöhle einführbar ist. Die Drainageleitung (8) ist mit dem erfindungsgemäßen Sensor versehen. In Flussrichtung nach dem Sensor ist ein Quetschventil (pinch valve, 11) geschaltet, welches die Drainageleitung (8) von der Saugpumpe entkoppelt. Ausgangsseitig der Saugpumpe (9) befindet sich noch ein Sammelbehälter (13).

In der erfindungsgemäßen Vorrichtung ist noch ein zweites ärztliches Instrument (6) vorhanden, beispielsweise ein Shaver. Bei Betrieb des zweiten ärztlichen Instrumentes (6) wird automatisch das Quetschventil (11) teilweise oder vollständig verschlossen, so dass der Volumenstrom des Fluids durch die Drainageleitung (8) zurückgeht. Der Drucksensor (10) misst zu diesem Zeitpunkt den Druck in der Körperhöhle (1). Wenn der Shaver ausgeschaltet wird öffnet sich automatisch das Quetschventil (11) so dass die Körperhöhle gespült wird. Gewünschtenfalls kann der Druck in der Körperhöhle während des Spülvorgangs durch den zweiten Drucksensor (12) gemessen werden.

### Beispiel

Die Genauigkeit der Messung im Rahmen dererfindungsgemäßen Vorrichtung wurde anhand eines Dummys bestimmt. Der Dummy enthält in der Körperhöhle einen Drucksensor, der den tatsächlichen Druck in der Körperhöhle anzeigt.

Die Druckmessung der erfindungsgemäßen Vorrichtung mit Drucksensor in der Drainageleitung (8) stimmt hervorragend mit dem tatsächlichen Druck überein sobald das Quetschventil geschlossen ist und der Fluss durch die Drainageleitung (8) auf Null zurückgeht. Bei geöffnetem Quetschventil unter Pumpenbetrieb ergeben sich naturgemäß höhere Pulsphasen in Abhängigkeit von der Peristaltik der Pumpe.

Die Verwendung der erfindungsgemäßen Vorrichtung hat zur Folge, dass Drucklimits wesentlich höher angesetzt werden können. Dies ermöglicht eine höhere Durchflussrate und damit bessere Spülbedingungen in der Körperhöhle mit insgesamt verbesserter Sicht.

Die erfindungsgemäße Vorrichtung weist einen zweiten Sensor (12) an der Druckseite der Spülpumpe auf. Dieser zweite Sensor kommt insbesondere dann zum Einsatz, wenn das Quetschventil geöffnet sein muss, z.B. während der Spülphasen im Rahmen von ärztlichen Behandlungen.

## Patentansprüche

1. Ansprüche (Juli 2014) :1. Vorrichtung zum Durchspülen einer Körperhöhle (1) mit einem Fluid, mit einer ansteuerbaren Spülpumpe (3) mit einer an die Druckseite der Spülpumpe (3) angeschlossenen Zuführleitung (4),
mit einem ersten ärztlichen Instrument (5),
mit einem zweiten ärztlichen Instrument (6), welches ein Shaver ist,
mit einer Drainagekanüle (7), angeschlossen an eine Drainageleitung (8) mit einem Quetschventil (11),
mit einer optionalen zweiten Pumpe (9) zum Absaugen des Fluids aus der Drainageleitung (8), und wobei ein zweiter Drucksensor (12) an der Druckseite der Spülpumpe (3) an der Zuführleitung angeschlossen ist, und **gekennzeichnet dadurch, dass** an der Drainageleitung (8) vor dem Quetschventil (11) ein erster Drucksensor (10) enthalten ist, der den Druck in der Körperhöhle (1) bestimmt und wobei
die Druckmessung mit dem ersten Drucksensor (10) erfolgt, wenn das Quetschventil (11) geschlossen ist und dass die Druckmessung mit dem zweiten Drucksensor (12) erfolgt, wenn das Quetschventil (11) offen ist.

2. Vorrichtung nach Anspruch 1, **dadurch gekennzeichnet, dass** das erste ärztliche Instrument (5) ausgewählt ist aus der Gruppe bestehend aus Spülsonde, Trokar mit -Optik, Optik mit Spülkanal, Spülkanüle, Shaver.

3. Vorrichtung nach Anspruch 1, wobei der Shaver 6 das Quetschventil (11) steuert, so dass bei Inbetriebnahme des Shavers, das Quetschventil in Stellung "zu" geht und bei Stillstand des Shavers das Quetschventil in Stellung "auf" geht.

## Claims

1. An apparatus for rinsing a body cavity (1) with a fluid, comprising a controllable rinse pump (3) with a supply line (4) connected the pressure side of the rinse pump (3),
comprising a first medical instrument (5), comprising a second medical instrument (6), which is a shaver,
comprising a drainage cannula (7), connected to a drainage line (8) with a pinch valve (11),
comprising an optional second pump (9) for sucking off the fluid from the drainage line (8), and a second pressure sensor (12) being connected at the pressure side of the rinse pump (3) to the supply line, and **characterized by** that a first pressure sensor (10) is included at the drainage line (8) upstream of the pinch valve (11), said first pressure sensor (10) determining the pressure in the body cavity (1), and wherein
the pressure measurement occurs with the first pressure sensor (10), when the pinch valve (11) is closed, and that the pressure measurement occurs with the second pressure sensor (12); when the pinch valve (11) is open.

2. The device according to claim 1, **characterized in that** the first medical instrument (5) is selected from the group consisting of rinse probe, trocar with optics, optics with rinse channel, rinse cannula, shaver.

3. The device according to claim 1, wherein the shaver (6) controls the pinch valve (11) such that when the shaver is put into operation, the pinch valve moves to position "closed", and when the shaver is at standstill, the pinch valve moves to position "open".

## Revendications

1. Dispositif de rinçage d'une cavité de corps (1) avec un fluide, comprenant une pompe de rinçage (3) commandable avec une conduite d'amenée (4) raccordée au coté extrados de la pompe de rinçage (3),
comprenant un premier instrument médical (5), comprenant un deuxième instrument médical (6), qui est un shaver,
comprenant une canule de drainage (7) raccordée à une conduite de drainage (8) avec une vanne à pincement (11),
comprenant une deuxième pompe (9) optionnelle pour la succion du fluide hors de la conduite de drainage (8), et un deuxième capteur de pression (12) étant raccordé au coté extrados de la pompe de rinçage (3) à la conduite d'amenée, et **caractérisé en ce qu'**un premier capteur de pression (10) est prévu à la conduite de drainage (8) en amont de la vanne à pincement (11), ce premier capteur de pression (10) déterminant la pression dans la cavité de corps (1), et dans lequel
le mesurage de pression a lieu avec le premier capteur de pression (10), si la vanne à pincement (11) est fermée, et que le mesurage de pression a lieu avec le deuxième capteur de pression (12), si la vanne à pincement (11) est ouverte.

2. Dispositif selon la revendication 1, **caractérisé en ce que** le premier instrument médical (5) est choisi à partir du groupe consistant en sonde de rinçage, trocart avec système optique, système optique avec canal de rinçage, canule de rinçage, shaver.

3. Dispositif selon la revendication 1, dans lequel le shaver (6) commande la vanne à pincement (11) de façon que, si le shaver est mis en fonction, la vanne à pincement soit dans la position «fermée», et si le shaver est à l'arrêt, la vanne à pincement soit dans la position «ouverte».
